# EUROPEAN PATENT APPLICATION

(11) **EP 2 567 687 A1**
(43) Date of publication of application: **13.03.2013**
(21) Application number: 11075208.6
(22) Date of filing: 12.09.2011
(51) Int. Cl.: A61J 1/20

(54) **Transfer device and transfer system for delivering a medical fluid to a medical fluid container**

(71) Applicant: BIRG Innovations Pty Ltd., Toowong QLD 4066 (AU)
(72) Inventor: Berrell, Ian, Camden NSW 2570 (AU)
(74) Representative: Pfenning, Meinig & Partner GbR

(57) **Abstract**

The present application relates primarily to a transfer device (1) for delivering a medical fluid to a medical fluid container (3), the transfer device (1) comprising at least
- a cannula (4) with a blunt tip (5) formed at a distal end (6) of the cannula (4) and with a side opening (7) arranged at an axial distance (8) from the blunt tip (5) and
- a retractable sheath (9), the sheath (9) being configured to repeatedly expose and seal the side opening (7),
wherein the distal end section of the sheath (9) has an open front surface (10) for passing through at least a part of the cannula (4).

The present application further relates to a transfer system comprising said transfer device and a bung for sealing a medical fluid container.

## Description

The subject matter of the present application primarily relates to a transfer device and to a transfer system for delivering a medical fluid to a medical fluid container.

Transfer systems for delivering a medical fluid, such as blood or liquid medication, to a medical fluid container, such as a blood collection tube or a vial, are well known from the prior art. Typically, such transfer systems comprise a bung for sealing the medical fluid container and a needle which is connected e.g. to a flexible tube from a winged set used for blood withdrawal from a patient. In order to deliver the medical fluid to the medical fluid container, the bung is pierced by the needle, thereby establishing a fluid connection between the needle and the medical fluid container. The delivery can be significantly facilitated if the medical fluid container is at least partially evacuated.

Known transfer systems suffer a number of disadvantages. In order for the needle to be capable of piercing the bung, a needle tip must be sufficiently sharp. However, sharp needles increase the risk of needle stick injury and dangerous infections on the part of a practitioner of the transfer device. Known transfer devices therefore shield the needle by means of a tube barrel holder into which the needle is incorporated. However, providing the tube barrel holder requires additional material and further production steps, thereby increasing production costs.

Thus, the objective of the present invention consists in the design of a transfer device for delivering a medical fluid to a medical fluid container, wherein the device
- minimizes the risk of needle stick injury,
- minimizes the probability of infections or poisoning due to exposure of a practitioner to the medical fluid when handling the device,
   and wherein
- costs for producing the device are significantly reduced with respect to known transfer devices.

This objective is achieved by a transfer device according to the main claim and by a transfer system comprising said transfer device. Special embodiments are described in the depending claims.

Thus, a transfer device for delivering a medical fluid to a medical fluid container is proposed, the transfer device comprising at least
- a cannula with a blunt tip formed at a distal end of the cannula and with a side opening arranged at an axial distance from the blunt tip and
- a retractable sheath, the sheath being configured to repeatedly expose and seal the side opening,
   wherein the distal end section of the sheath has an open front surface for passing through at least a part of the cannula.

Within the scope of this document, the term "blunt" used in connection with the tip of the cannula implies that the tip is not capable of inflicting damage on a living human's skin, e.g. the skin of an arm, so long as a force exerted on the skin through the tip is smaller than ten, preferably as long as the force is smaller than twenty Newtons (i.e. smaller than a gravitational force acting on a body with a mass of one or two kilograms, respectively). When testing the tip's bluntness in this way, the force exerted on the skin is at least 0.1 Newton, preferably at least 1 Newton, more preferably at least 5 Newtons. In particular, the tip is not capable of piercing the skin when the force is smaller than the cited value and applied at a right angle with respect to the skin. Thus, owing to the blunt tip forming the distal end of the cannula, the transfer device is particularly safe to use as there is virtually no risk of the cannula piercing or scratching a practitioner's skin when the cannula is handled normally. Hence, a danger of the practitioner suffering needle stick injury or infections as a result of unwanted contact with the medical fluid is significantly reduced.

The side opening of the cannula establishes a fluid connection between an interior lumen of the cannula and the cannula's exterior. The sheath can cover or enclose the cannula at least partially. When the sheath exposes the side opening, i.e. when the sheath is in an open state, the medical fluid is capable of exiting the interior lumen through the side opening. On the other hand, when the sheath seals the side opening, i.e. when the sheath is in a closed state, the sheath stops a flow of the medical fluid through the side opening. Preferably, when in the closed state the sheath covers the side opening and is in direct contact with a section of a surface of the cannula enclosing the side opening. I.e. in the closed state the sheath advantageously prevents unwanted spill of the medical fluid through the side opening, thereby increasing the safety of the transfer device and making it more clean and comfortable to use.

The cannula axis defines a z-axis of a right-handed Cartesian coordinate system in which the cannula is permanently at rest, an x-axis and a y-axis being perpendicular to the z-axis. A positive z-direction points along the cannula axis towards the blunt tip. Typically, in order to expose the side opening the sheath is configured to be pushed or slid at least partially over the side opening in the negative z-direction. For sealing the side opening, the sheath can be pushed or slid at least partially over the side opening in the positive z-direction.

Preferably, the sheath can be readily moved, slid or pushed over the side opening of the cannula by hand, e.g. by inserting the cannula in a correspondingly formed opening of the medical container into which the medical fluid is to be delivered, the opening of the medical container preferably being configured to accommodate the cannula but not the sheath. Typically, the cannula has an axial length of a few centimetres, preferably of at least one centimetre, wherein the axial length is determined along the z-axis. Preferably, the axial distance between the side opening and the blunt tip is a few millimetres, preferably at least two millimetres, most preferably at least four millimetres. A circumference of the cannula at a z-position of the side opening can amount to at least two millimetres, preferably to at least four millimetres, more preferably to at least eight millimetres, the circumference being measured in the x-y-plane. The side opening may have a circular shape with a diameter of between 0.1 mm and 3 mm, preferably between 0.1 mm and 1 mm.

The open front surface of the sheath, in the following also termed front opening or front hole, is a permanent open hole or perforation of the sheath at a distal end of the sheath. In the closed state of the sheath, the front opening of the sheath is typically arranged at an axial distance from the side opening of the cannula along the positive z-direction. By contrast, in the open state of the sheath, the front opening of the sheath is typically arranged at an axial distance from the side opening of the cannula along the negative z-direction. I.e. as the sheath is brought from the closed state to the open state and back an axial section of the cannula comprising the side opening is passed through the front opening of the sheath. In other words, the sheath can be brought to the open and/or to the closed state by moving or sliding its distal end or a distal end section of the sheath over the side opening.

In particular, the front opening is configured as a non-closed hole both in the open and in the closed state of the sheath, meaning that the sheath is not pierced or destroyed upon exposing the side opening. This is in contrast to elastic rubber coverings for sharp needles known from the prior art. These coverings have a closed front surface which is pierced, i.e. "opened" upon a first use of the sharp needle. Therefore, needles known from the prior art featuring said coverings exhibit an acuate tip in order to "open" the covering by piercing through it.

Thus, in the present invention the advantageous combination of the blunt cannula tip and the re-sealable side opening is realized by the side opening's being axially spaced from the cannula tip, by the sheath's featuring the front opening and by the front opening's passing through at least part of the cannula upon exposing and sealing the side opening. By virtue of the open front surface of the sheath the cannula tip need not pierce the sheath in order to expose the side opening, thereby allowing the tip to be blunt. Since the side opening is arranged on a side of the cannula rather than at its tip, the sheath can effectively seal and expose the side opening without being pierced or destroyed.

Usually, the cannula additionally features a second opening, the side opening being arranged in between the second opening and the blunt tip along the z-axis. Then, also the second opening is in fluid communication with the central lumen of the cannula, allowing the medical fluid to enter the central lumen through the second opening, to flow through the central lumen and to exit the central lumen through the side opening or vice versa. The second opening can serve as a port or adapter through which the medical fluid can be delivered to the cannula from a medical fluid conductor such as a pipe, a flexible tube, a syringe or the like. In other words, the second opening can be configured to be connected to a tube, e.g. to a butterfly tubing of a winged needle set, or to a syringe. Through the side opening, the medical fluid can be delivered to the medical fluid container or conductor, which can be an at least partially evacuated tube, preferably a blood collection or storage tube, a blood bag, a vial, a syringe, a flexible tube, a rigid pipe or a similar container or conductor. The medical fluid can comprise blood, other body liquids or liquids used for medical treatment such as liquid medication.

In a special embodiment, the cannula comprises a plastic material. The plastic material can account for at least 80 percent, preferably for at 90 percent, more preferably for at least 95 percent by weight of the cannula. The cannula can also be entirely made of the plastic material. Preferably, at least a distal section of the cannula is entirely made of the plastic material, the distal section of the cannula preferably extending at least from the tip to the side opening, more preferably beyond the side opening in the negative z-direction, e.g. by at least 3 mm, by at least 5 mm or by at least 1 cm. The plastic material can comprise polypropylene (PP), polycarbonate (PC), acrylonitrile butadiene styrene (ABS) or mixtures thereof. It can also comprise other plastic materials or mixtures of other plastic materials. Plastic materials are easy to shape, can be produced at particularly low cost and may be disposed cheaply and easily. Furthermore, plastic materials have a low weight while at the same time exhibiting a sufficient degree of stiffness and rigidity. Plastic cannulas significantly reduce a risk of injury for the practitioner.

In a further special embodiment, the sheath comprises an elastic material, e.g. a thermoplastic elastomer. A Shore A hardness of the material of the sheath can be between 40 and 80. Owing to its elasticity, the sheath can clasp around the cannula and can be easily capable of folding as it is moved from the closed state to the open state. Preferably, the sheath firmly clasps around the cannula at least partially in the open and/or in the closed state of the sheath. Also, the elastic sheath can at least partially adapt to a varying circumference of the cannula along the cannula axis. Furthermore, identical elastic sheaths can be used in connection with slightly differently shaped cannulas. Due to the sheath's elasticity, the sheath and the cannula can be frictionally connected at least partially. The elasticity of the sheath can allow for an area of the front opening of the sheath to be slightly smaller than a cross-sectional area of the cannula at the z-position of the side opening. Alternatively, the sheath can be designed e.g. as a rigid hollow cylinder.

In a further special embodiment the sheath has a tubular or hose-like shape, the sheath thereby enclosing the cannula at least partially. In other words, the sheath can form or enclose a central lumen, whereby a cross-sectional area of this lumen can have a shape which is anywhere between circular and slot-like. Typically, the lumen's cross-sectional area is elliptical or nearly elliptical. In this manner, the sheath can effectively accommodate the cannula, the sheath and the cannula having corresponding shapes at least along axial sections of the sheath and the cannula. At a proximal end or at a proximal section of the sheath, i.e. at an end or at a section of the sheath averted from the distal end of the sheath, the sheath and the cannula can be permanently frictionally connected. Likewise, the sheath and the cannula can be in a form-fit or they can be welded, melted or glued to each other at the proximal end of the sheath.

In a further special embodiment, a mechanical stop arranged at or connected to the cannula is configured to prevent the sheath from being pushed beyond the mechanical stop along the z-direction, typically along the negative z-direction. Preferably, the mechanical stop is arranged at an axial distance from the side opening towards the negative z-direction. In other words, the side opening is usually arranged, along the z-axis, in between the mechanical stop and the cannula tip. The mechanical stop can be designed as one or several protrusions sticking out from the cannula in the x-y-plane. Preferably, the mechanical stop is designed as a handle at which the practitioner can hold the transfer device. In a preferable design the mechanical stop has the shape of flat winged extensions projecting from opposite sides of the cannula.

In a further special embodiment, a stiffness and/or an elasticity of the sheath causes the sheath to seal the side opening when no external forces act on the sheath. In particular, the sheath can push itself off the mechanical.stop, thereby springing back or pushing back over the side opening in the absence of external forces. In other words, the sheath can be configured to automatically seal the side opening when no external forces act on the sheath, e.g. when the cannula is removed from the medical fluid container after delivery of the medical fluid. This implies that the side opening can be sealed unless a small opening force is exerted on the sheath to bring it to the open state.

Owing to this feature, spill of the medical fluid is effectively prevented. Furthermore, the practitioner saves an additional work step which would otherwise be needed to seal the side opening. This is a significant advantage over transfer devices known from the prior art which require that the practitioner open and close a clipping element each time he intends to stop or to allow a flow of the medical fluid through a tube feeding the cannula. Typically, the sheath is brought to the open state by sliding, through the application of the small opening force, the distal section of the sheath in the negative z-direction over the side opening. The proximal section of the sheath is then kept in place by e.g. the mechanical stop, causing at least a central section of the sheath to fold and/or bend and/or distort. With no opening force present, the stiffness of the sheath then causes the distal section of the sheath to slide back over the side opening in the positive z-direction, thereby re-sealing the side opening.

In a further special embodiment, the sheath has perforations, the perforations allowing the sheath to fold at least partially when the sheath exposes the side opening. By removing material from the sheath in the perforated regions, the folding of the sheath for bringing the sheath to the open state is greatly facilitated. Typically, the perforations are designed as slits or holes arranged along the z-axis. By introducing the perforations in the sheath, the stiffness of the sheath can be manipulated.

In a further special embodiment, the cannula has a non-circular cross section at least in a distal section of the cannula wherein the distal section preferably comprises the side opening of the cannula. This can greatly facilitate inserting the cannula in a corresponding opening of e.g. the medical fluid container into which the medical fluid is to be delivered by reducing a frictional force between the cannula and the corresponding opening of the container. Preferably, the cross-sectional area of the cannula in the distal section of the cannula has an elliptical or nearly elliptical shape. A ratio of a length of a long axis of an ellipsis over a short axis of the ellipsis can be at least two to one, preferably at least three to one, more preferably at least four to one. In the distal section of the cannula, the cannula typically tapers towards the blunt tip. At least in one lateral direction, i.e. in a direction perpendicular to the cannula axis or z-axis, a radius of curvature of the cannula at the tip is at least 0.5 mm, preferably at least 1 mm, more preferably at least 2 mm or at least 3 mm. Preferably, a radius of curvature at the tip is larger than the aforementioned values in all lateral directions. The tip usually has an at least partially spherical or rounded shape.

If the cannula is made of a plastic material, the tip can be "blunt" according to the definition given above even when featuring an edge. E.g. the distal section of the cannula can feature an oval or elliptical cross section, the x-axis being the long axis and the y-axis being the short axis of the ellipsis. In this case a radius of curvature of the tip in the y-direction can be small, e.g. the radius of curvature in the y-direction ranging between 0.3 mm and 1 mm, thereby forming an edge at the tip of the cannula running in the x-direction. As long as the radius of curvature at the tip in the x-direction is at least 1 mm, preferably at least 2 mm, more preferably at least 3 mm, the overall shape of the tip can still be "blunt" according to the definition given above.

In a further special embodiment, a cross-sectional area of a central lumen of the cannula amounts to less than 30 percent, preferably to less than 20 percent, more preferably to less than 10 percent of a cross-sectional area of the cannula, at least in the distal section of the cannula, i.e. at least in an axial section of the cannula extending at least from the tip to the z-position of the side opening. This way, a stability of the cannula is advantageously improved. The central lumen can have a circular or non-circular cross-sectional area. A diameter of the cross-sectional area of the central lumen, or a long axis of the cross-sectional area of the central lumen in the case that the cross sectional-area of the central lumen is non-circular, can be at least 0.2 mm, preferably at least 0.5 mm. Typically, the diameter or the long axis of the cross-secional area of the central lumen is smaller than 2 mm, preferably smaller than 1 mm or than 0.8 mm.

In a further special embodiment, the cannula is at least partially coated with a lubricating coating, e. g. with silicone. The lubricating coating protects an outer surface of the cannula and increases a smoothness of the cannula's outer surface. In this manner, the cannula can be more easily inserted into an opening of the medical container by reducing the friction between a material enclosing the opening of the medical container and the outer surface of the cannula. Also, friction between the cannula surface and the sheath is minimized, allowing the sheath to be slid over the cannula surface more easily. Alternatively, the cannula can be made of Polypropylene. Polypropylene is self lubricating and thus has a similar effect as the aforementioned lubricating coating.

In a further special embodiment, the aforementioned second opening of the cannula is configured as a male or a female part of a Luer Taper connection. In other words, the second opening is configured to be connected to a male or a female part of a Luer Taper connection or to components featuring a male or a female part of a Luer Taper connection, such as a syringe or a butterfly needle tubing of a winged needle set.

Also, a transfer system is proposed, the transfer system comprising a transfer device according to the preamble of the main claim and the transfer system further comprising a bung for sealing the medical fluid container, wherein the sheath is configured to expose the side opening upon insertion of the cannula into an opening of the bung and to reseal the side opening upon withdrawal of the cannula from the opening of the bung.

It should be noted that no protection is sought for the medical fluid container. The scope of protection is merely intended to cover the transfer device and the bung.

The bung can have a cylindrical or at least partially cylindrical or at least partially conical shape and/or comprise a rubber material, e.g. a thermoplastic elastomer such as thermoplastic polyurethane. The opening of the bung is typically configured to accomodate at least the distal section of the cannula and to push or slide the sheath in the negative z-direction as the cannula is inserted into the opening of the bung. I.e. upon insertion of the cannula into the opening of the bung, a material of the bung enclosing the opening of the bung pushes or slides the sheath into the open state, thereby exposing the side opening of the cannula and allowing the medical fluid to be drawn or to be delivered into the medical container sealed by the bung. In other words, upon insertion of the cannula into the opening of the bung, the side opening of the cannula is inserted all the way into an interior of the medical fluid container, thereby establishing a fluid connection between the interior of the medical fluid container and the central lumen of the cannula. Due to the fact that the sheath of the transfer device is configured to expose and to seal the side opening repeatedly, e.g. at least 8, preferably at least twenty, more preferably at least fifty times, the presently claimed transfer device and transfer system feature a performance which is superior to known transfer systems and transfer devices and is in compliance with existing regulations which require that a needle endure 4 to 8 insertions into a medical fluid container.

In a further special embodiment, the transfer device is incorporated into or connected to a tube barrel holder, the tube barrel holder being configured to accommodate the fluid container at least partially and to guide the cannula into the opening of the bung. The cannula and the holder can be made of the same material and/or be configured as a single piece. Typically, the holder features a cavity which is open at a distal end of the holder, the medical container being configured to be inserted into the holder at least partially through the open distal end of the holder. The transfer device can be incorporated at a proximal end of the holder, the proximal end of the holder being averted from the distal end of the holder. Typically, the holder has a shape of a hollow cylinder which is partially closed at the proximal end of the holder, the proximal end of the holder thereby serving as a mechanical stop for the bung upon insertion of the cannula into the bung.

In a further special embodiment, the opening of the bung is designed as a self-resealable and non-circular slit, the slit being configured to accommodate the cannula at least partially and the bung preferably tightly clasping around at least a part of the cannula upon insertion of the cannula into the slit. With the opening of the bung being configured as a slit, a force required to insert the cannula into and to withdraw it from the opening of the bung is reduced. The slit can completely perforate the bung. Alternatively, the slit can perforate the bung only partially, whereby the slit is fully perforated upon insertion of the cannula into the bung. The bung can feature additional cavities or recesses, the cavities or recesses being configured to be at least partially compressed upon insertion of the cannula into the slit, thereby further reducing a force required for inserting the cannula into the slit or for withdrawing it from the slit.

In a further special embodiment, the sheath comprises protuberances at the distal end of the sheath thereby increasing a contact surface between the sheath and the bung during insertion of the cannula into the opening of the bung, the bung preferably having corresponding recesses for accommodating the protuberances. Owing to the protuberances, the sheath can be brought to its open position more easily upon insertion of the cannula into the opening of the bung. Typically, the protuberances extend laterally from the sheath by e.g. between 0.5 mm and 2 mm.

The invention will now be described by way of example with reference to the accompanying drawings in which
- Fig. 1: shows a cannula of a transfer device,
- Fig. 2: shows a sectional view of the cannula,
- Fig. 3: shows a y-z-section of a distal end of the cannula,
- Fig. 4: shows a x-z-section of the distal end of the cannula,
- Fig. 5: shows a x-y-section of the distal end of the cannula,
- Fig. 6: shows a perspective view of a transfer device comprising the cannula and a retractable sheath, the heath sealing a side opening of the cannula,
- Fig. 7: shows a sectional view of the transfer device depicted in Fig. 7,
- Fig. 8: shows a sectional view of the transfer device with the sheath exposing the side opening,
- Fig. 9: shows a sectional view of a transfer system comprising the transfer device and a medical fluid container sealed by a slit bung,
- Fig. 10: shows a sectional view of the transfer system, the cannula being partially inserted into the bung,
- Fig. 11: shows a detailed view of the bung,
- Fig. 12: shows the transfer system additionally comprising a tube barrel holder.
- Fig. 13: shows a perspective view of the transfer device, wherein a proximal opening of the cannula is configured as a female part of a Luer Taper connection,
- Fig. 14: illustrates a sectional view of the transfer device shown in Fig. 13, and
- Fig. 15: shows the transfer device shown in Figs. 13 and 14 being connected to a syringe with a male part of a Luer Taper connection.

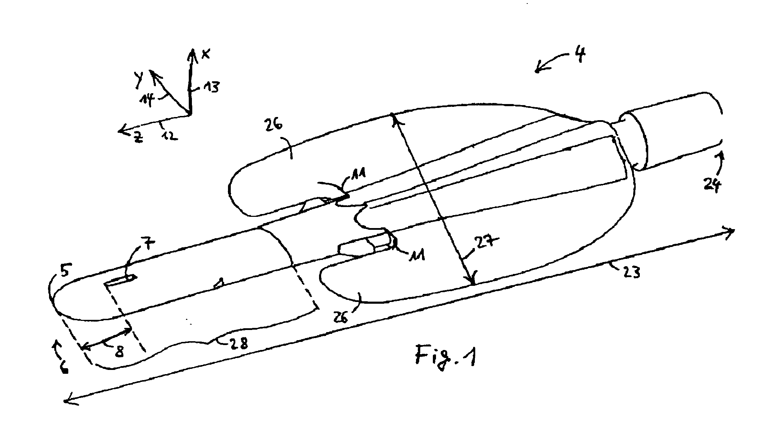

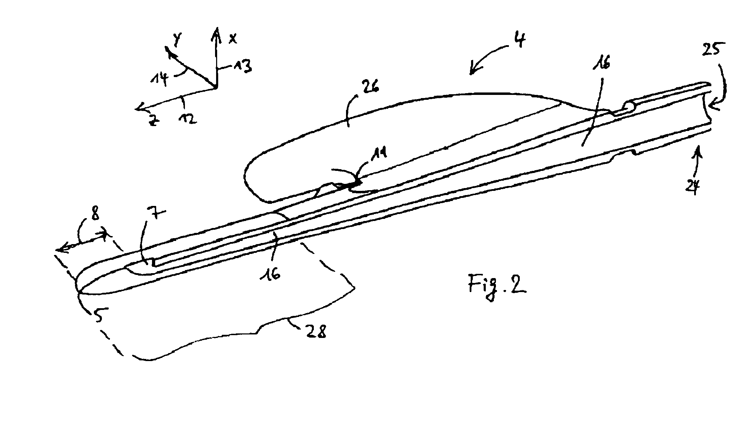

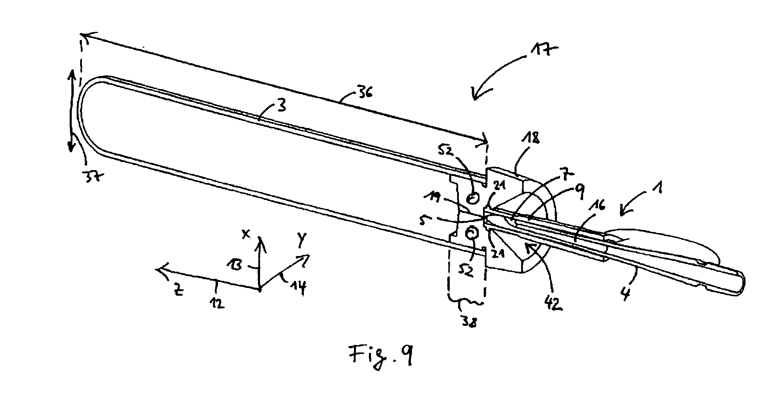

Figs. 1 and 2 illustrate an elongate cannula 4 extending over a length 23 of 7 cm from a distal end 6 to a proximal end 24. Here and in all of the following recurring features are designated with identical reference numbers. The cannula 4, which is entirely made of polycarbonate, is designed to deliver blood to a partially evacuated blood collection tube (Fig. 9). A flexible tube of a winged needle set (not shown) used for blood withdrawal from a patient feeds the blood via a proximal opening 25 at the proximal end 24 of the cannula 4 into a central lumen 16 of the cannula 4, the central lumen 16 extending over almost the entire length 23 of the cannula 4. From the central lumen 16 the blood can exit the cannula 4 via a side opening 7 which is arranged at a side of a distal section 28 of the cannula 4. The side opening 7 establishes a fluid communication between the central lumen 16 and an exterior of the cannula 4.

In a reference frame of the cannula 4 a longitudinal direction of the cannula 4 defines a z-axis 12 of a right handed Cartesian coordinate system, the positive z-direction pointing from the proximal end 24 towards the distal end 6 of the cannula 4. The longitudinal direction is also referred to as an axial direction. An x-axis 13 and a y-axis 14 define a lateral x-y-plane. At the distal end 6 the cannula 4 forms a blunt safety tip 5, the tip 5 not being capable of piercing a human's skin when handled normally, i.e. when a force exerted on the skin through the tip 5 of the cannula 4 at a right angle is smaller than 20 N. The side opening 7 is arranged at an axial distance 8 of 5 mm from the tip 5. In a central section of the cannula 4 flat winged extensions 26 project from opposite sides of the cannula 4 over a maximum lateral extension 27 of 3 cm within the y-z-plane, the extensions 26 serving as a handle for a practitioner. Protrusions of the extension 26 facing the distal end 6 of the cannula 4 form a mechanical stop 11 of the cannula 4. A function of the mechanical stop 11 is described further below. In the present embodiment, the cannula 4 is additionally coated with silicone (not shown) in order to enhance a slippage of a surface of the cannula 4.

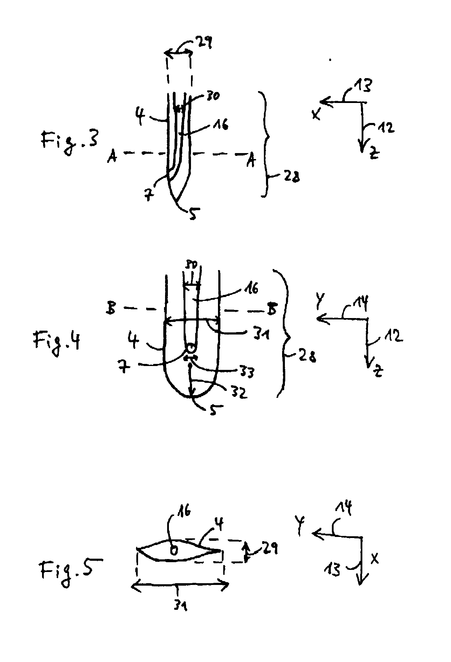

Figs. 3, 4 and 5 show details of a shape of the cannula 4 in the distal section 28 of the cannula. Fig. 3 shows an x-z-section of the distal section 28. From the side opening 7 upward, i.e. towards the negative z-direction, the distal section 28 of the cannula 4 has an x-thickness 29 of 2.5 mm. A diameter 30 of the central lumen 16 is slightly increasing towards the negative z-direction, the diameter 30 being smaller than 1 mm from the side opening 7 upwards throughout the distal section 28 of the cannula 4. From the side opening 7 downwards, i.e. towards the tip 5, the cannula 4 tapers at an angle of roughly 30 degrees with respect to the axial direction. At the tip 5 a radius of curvature (not shown) of the tip 5 in the x-z-plane is about 0.5 mm. Thus, the tip 5 features a relatively sharp edge running in the y-z-plane. However, due to a substantially larger radius of curvature of the tip in the y-z-plane (Fig. 4) and owing to the fact that the cannula 4 is made of plastic, the tip 5 is still blunt according to the definition given above (no piercing of a human's skin at a force smaller than 20 N).

Fig. 4 depicts a y-z-section of the distal section 28 of the cannula 4. Above the side opening 7 in Fig. 4 the cannula 4 features a y-thickness of 6 mm. From the side opening 7 downward, i.e. towards the tip 5, the cannula 4 tapers almost along a circular line, a radius of curvature 32 of the tip 5 in the y-z plane being 3 mm. The diameter 30 of the central lumen 16 increases slightly from the side opening 7 upwards, but remains smaller than 1 mm throughout the distal section 28 of the cannula 4. A diameter 33 of the circular side opening 7 at a surface of the cannula 4 is 0.8 mm.

Fig. 5 shows an x-y-section of the cannula 4 along the lines A-A in Fig. 3 and B-B in Fig. 4. It can be clearly observed that a cross-sectional area of the cannula 4 in the distal section 28 is non-circular. More specifically, the cross-sectional in Fig. 5 has a nearly oval shape. Furthermore, in the sectional view of the distal section 28 of the cannula 4 depicted in Fig. 5, a cross-sectional area of the central lumen 16 accounts for less than 10 percent of the cross-sectional are of the cannula 4, lending the cannula 4 a high degree of mechanical stiffness and stability.

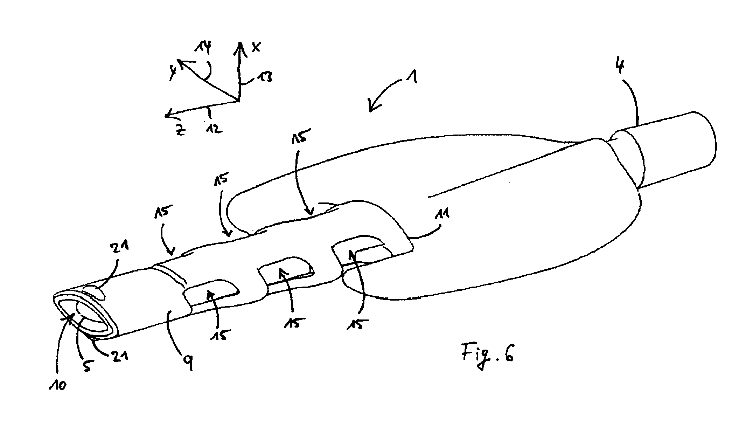

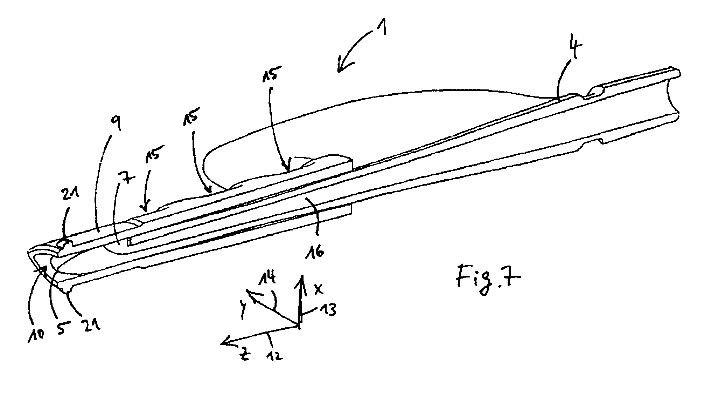

Figs. 6 and 7 show a perspective view and a sectional view, respectively, of the cannula 4. In between the distal end 6 of the cannula 4 and the mechanical stop 11 the cannula 4 is enclosed by a hose-like or tubelike sheath 9. The sheath 9 is entirely made of an elastic thermoplastic elastomer with a Shore A hardness of 60. A thickness of the sheath 9 is about 1 mm. The cannula 4 and the sheath 9 form a transfer device 1.

In Figs. 6 and 7 the sheath 9 tightly clasps around the cannula 4, thereby firmly sealing the side opening 7. I.e. in Figs. 6 and 7 blood contained within the central lumen 16 is prevented from exiting the cannula through the side opening 7 by the sheath 9. In the closed position of the sheath 9 shown in Figs. 6 and 7, the sheath 9 is in a weak frictional connection with the cannula 4 by virtue of its elasticity. In particular, in the closed position of the sheath 9 depicted here, the sheath is in direct contact with a part of an outer surface of the cannula 4 enclosing the side opening 7 at the outer surface of the cannula 4. At a distal end of the elastic sheath 9 the sheath 9 features protuberances 21 sticking out on opposite sides of the sheath at right angles by approximately 0.8 mm.

Notably, in the closed state of the tubular elastic sheath 9 shown here, the sheath 9 has an open front surface 10 at its distal end. From Figs. 6 and 7 it can be clearly seen that the open front surface 10 constitutes a permanent opening or perforation of the sheath 9 at its distal end. Due to its elasticity, the sheath 9 adjusts its shape to the cannula 4. Therefore, a cross-sectional area of the open front surface 10 of the sheath matches the cross-sectional area of the cannula 4 at a z-position of the side opening 7. In fact, in Figs. 6 and 7 the cross-sectional area of the front opening 10 can be slightly smaller, e.g. five percent smaller, than the cross-sectional area of the cannula 4 at the z-position of the side opening 7 from where on the cannula 4 tapers towards the tip 5.

Furthermore, the sheath 9 features perforations 15 arranged sideways on opposite sides of the sheath 9. On each side, three elongate perforations 15 are aligned along the axial direction. The perforations 15 reduce a mass of the sheath 9 by about five percent. Additionally, the perforations 15 reduce a stiffness of the sheath 9, i.e. a resistance of the sheath 9 to deformations of the sheath 9 along the z-axis 12.

Importantly, no external forces are acting on the sheath 9 in Figs. 6 and 7. In other words, despite the perforations 15 the stiffness of the sheath 9 causes the sheath 9 to assume its elongate equilibrium hose-like shape, thereby covering and sealing the side opening 7 and preventing a spill of blood from the lumen 16.

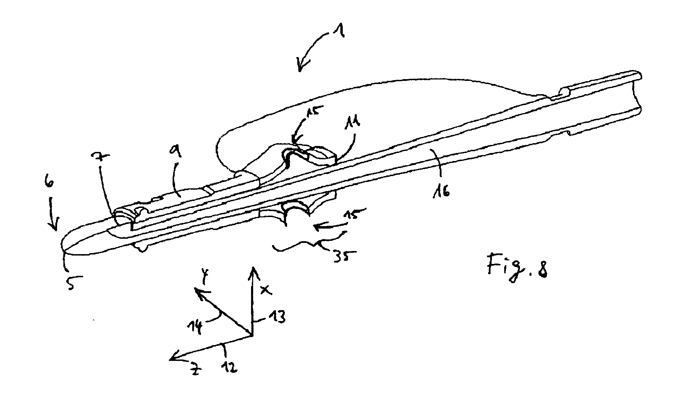

In contrast to Figs. 6 and 7, Fig. 8 shows a sectional view of the transfer device 1 in which the sheath 9 no longer seals the side opening 7. Rather, the sheath is pushed or slid over the side opening 7 in the negative z-direction, i.e. away from the distal end 6 and towards the proximal end 24 of the blunt plastic cannula 4. In other words, in the open state or open position of the sheath 9 depicted in Fig. 8, the sheath 9 exposes the side opening 7, thereby allowing blood contained in the central lumen 16 of the cannula 4 to exit the cannula 4 through the side opening 7. The sheath 9 is brought from the closed position shown in Figs. 6 and 7 to the open position illustrated in Fig. 8 by application of a small opening force. Thus, in Fig. 8 an opening force acts on the sheath 9.

Due to the perforations 15 the sheath 9 is allowed to fold at least in a proximal section 35 of the sheath as it is retracted from the side opening 7 and as it exposes the side opening 7. In other words, the perforations 15 facilitate an opening movement or a retraction movement of the sheath 9 from the closed position to the open position of the sheath 9. During the opening movement the mechanical stop 11 prevents the sheath 9 from being pushed or slid beyond the mechanical stop 11 in the negative z-direction.

If the sheath 9 is no longer pushed or pressed against the mechanical stop 11 by the opening force, its stiffness causes the sheath 9 to relax and to assume its equilibrium shape. I.e. from the open position the sheath 9 automatically folds back to the closed position if no more external force acts on it. In the course of this process the distal end of the sheath 9 is again pushed towards the tip 5 of the cannula 4 and over the side opening 7, thereby sealing the side opening 7. In the embodiment of the transfer device 1 described here, the sheath is configured to seal and to expose the side opening 7 repeatedly, e.g. more than 20 times, before the elastic material of the sheath 9 undergoes any noticeable fatigue. In the course of the opening and closing movement of the sheath 9, a part of the distal section 28 of the cannula 4, namely a part of the distal section 28 comprising the side opening 7, is repeatedly passed through the open front area 10 of the sheath 9 in the positive and the negative z-direction. By improving the slippage of the cannula surface the cannula's silicone coating facilitates the opening and the closing of the sheath 9. In other words, the silicone coating of the cannula 4 reduces a friction between the cannula 4 and the sheath 9 as it is brought from the closed position to the open position and vice versa.

Figs. 9 through 12 show a transfer system 17 which includes the transfer device 1 and a rubber bung 18, the bung 18 sealing a medical fluid container 3. In the embodiment illustrated in Figs. 9 through 12 the medical fluid container 3 is configured as a partially evacuated blood collection tube which is made of a transparent plastic material. Alternatively, the medical fluid container 3 can be made of glass or non-transparent plastic materials. The cylindrically shaped medical fluid container 3 has a length 36 of 120 mm, a diameter 37 of 15 mm and a wall thickness of 1 mm (not shown).

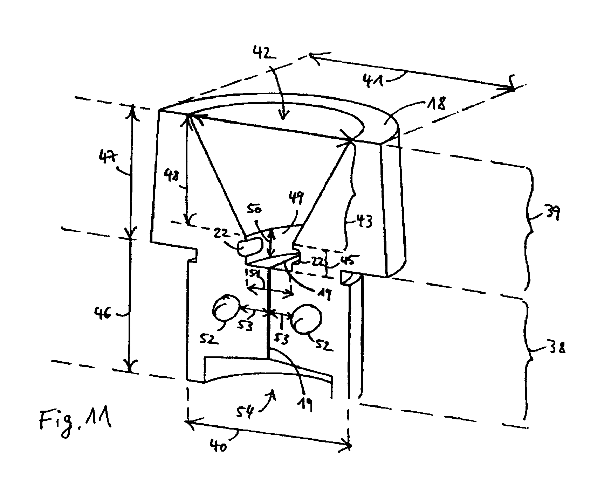

The rubber bung 18 sealing the medical fluid container 3 in Fig. 9 is depicted in detail in Fig. 11. The bung 18 forms a cylindrically shaped lower section 38 having a length 46 of 8 mm and an upper section 39 having a length 47 of 10 mm. The lower section 38 and the upper section 39 of the bung 18 are made of one piece of elastic rubber. A diameter 40 of the lower section 38 is 13 mm, the lower section 38 thereby being configured to be inserted into an opening of the medical fluid container 3, as shown in Figs. 9, 10 and 12. A diameter 41 of the upper section 39 of the bung 18 is 17 mm.

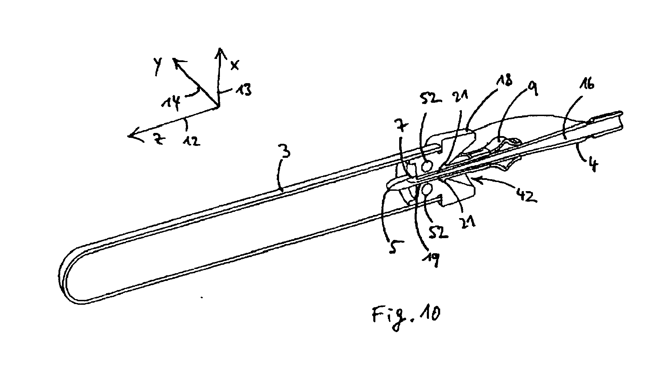

A recess 42 formed within the bung 18 comprises a conically shaped first sector 43 with a height 48 of 8 mm, the first sector 43 tapering from a circular upper end with a diameter 44 of 13 mm to an oval-shaped lower end. At the lower end, the first sector 43 of the recess 42 passes into a second sector 45 of the recess 42. The second sector 45 has an oval-shaped x-y-cross section and is enclosed by a vertical side wall 49 with a height 50 of 1 mm. A short axis of the oval-shaped cross section of the second sector 45 has a length 51 of 3.5 mm while a long axis of the second sector 45 has a length of 8 mm (not shown). The vertical wall 49 of the second sector 45 further comprises recesses 22. These are configured to accommodate the protuberances 21 extending from the distal end of the sheath 9 as the transfer device 1 is inserted into the bung 18 through the recess 42 (Fig. 9 and 10).

An opening 19 in the bung 18 is shaped as a non circular slit extending in the y-z-plane. The opening 19 connects the second sector 45 of the recess 42 to an underside 54 of the bung 18 and is configured to accommodate at least the distal section 28 of the cannula 4. Cavities 52 arranged in the lower section 38 of the bung 18 at lateral distances 53 from the slit-like opening 19 further facilitate the insertion of the cannula 4 into the opening 19. As long as the cannula 4 is not inserted into the opening 19, however, a stiffness of the rubber bung 18 causes the opening 19 to seal.

Figs. 9 and 10 demonstrate the insertion of the cannula 4 into the bung 18. In Fig. 9 the sheath 9 is in the closed position, enclosing and clasping the cannula 4. Thus, the sheath 9 seals the side opening 7 of the cannula 4, preventing blood inside the central lumen 16 of the cannula 4 to pass through the side opening 7. The transfer device 1 has been inserted into the recess 42 of the bung 18, the tip 5 of the cannula 4 and the distal end of the sheath 9 being in contact with the lower section 38 of the bung 18. In this position, the protuberances 21 of the sheath 9 have been brought into a form-fit with the recesses 22 in the side wall 49 of the second sector 45 of the recess 42 of the bung 18 (see also Fig. 11). The stiffness of the rubber bung 18 and the partial vacuum within the medical fluid container 3 cause the slit-like opening 19 in the lower section 38 of the bung 18 to close, thereby sealing the medical fluid container 3.

Fig. 10 shows the transfer system 17 in an arrangement where the cannula 4 has been partially inserted and pushed through the opening 19 of the bung 18. Insertion of the cannula 4 into the bung 18 is facilitated by the taper of the of the cannula 4 towards the tip 5 (Fig. 3), by the silicone coating of the cannula surface which reduces friction between the cannula surface and the bung opening 19 and by the cavities 52 which are slightly compressed as the opening 19 accomodates the cannula 4. Friction between the opening 19 and the cannula 4 is further reduced due to the oval cross section of the cannula 4. Since the sheath 9 is not configured to be accommodated by the opening 19 of the bung, the sheath 9 is held back by a part of the bung 18 enclosing the opening 19 as the distal section 28 of the cannula 4 is inserted into the opening 19. In the course of this process, an opening force is exerted on the sheath 9, causing it to fold at least partially and to retract from the side opening 7, thereby passing at least a part of the cannula 4 through the open front surface 10 of the sheath. The mechanical stop 11 prevents the sheath 9 from being pushed beyond the mechanical stop 11 in the negative z-direction, i.e. along the cannula axis.

In the arrangement of the transfer system 17 shown in Fig. 10, the distal section 28 of the cannula 4 has been inserted into and through the opening 19 of the bung 18 such that the opening 7 of the cannula 4 is situated below the underside 54 of the bung 18, allowing blood from the central lumen 16 of the cannula 4 to be drawn into the blood collection tube, i.e. into the medical fluid container 3. Retraction of the sheath 9 from the side opening 7 during the insertion of the cannula 4 is facilitated by the recesses 15 of the sheath 9 (not shown in Fig. 10 for simplicity), by the silicone coating of the cannula surface and by the engagement of the protuberances 21 of the sheath 9 in the recesses 22 of the bung 18. While the recesses 15 of the sheath 9 and the silicone coating of the cannula surface reduce a resistance of the sheath 9 against folding and minimize friction between the sheath 9 and the cannula 4, respectively, the engagement of the protuberances 21 in the recesses 22 increases a contact surface between the sheath 9 and the bung 18. During insertion of the cannula 4 into the bung 18, the latter firmly clasps around the cannula 4, thereby still sealing the partially evacuated medical fluid container 3.

As the cannula 4 is withdrawn from the bung 18, the stiffness of the sheath 9 causes the sheath 9 to fold back to its unfolded equilibrium position and to reseal the side opening 7. In this manner, spill of blood upon withdrawal of the transfer device 1 from the bung 18 is advantageously avoided.

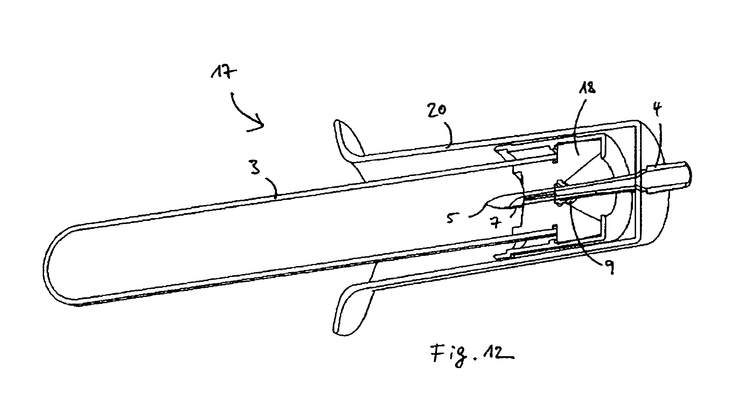

Fig. 12 shows an alternative embodiment of the transfer system 17. In this embodiment, the cannula 4 of the transfer device 1 is incorporated into a tube barrel holder 20. The cannula 4 and the tube barrel holder 20 have been shaped from the same piece of plastic material. The barrel holder 20 has the form of a hollow cylinder which is partially closed on one end. At an open end of the hollow cylinder the tube barrel holder 20 is configured to partially accommodate the medical fluid container 3, the container 3 and the holder 20 having corresponding shapes. The tube barrel holder 20 further protects the practitioner from the cannula 4, in particular from the tip 5 of the cannula 4. Also, the tube barrel holder 20 facilitates guiding the cannula 4 into the opening 19 of the bung 18 and allows for one-handed manipulation of the cannula 4 into the medical fluid container 3.

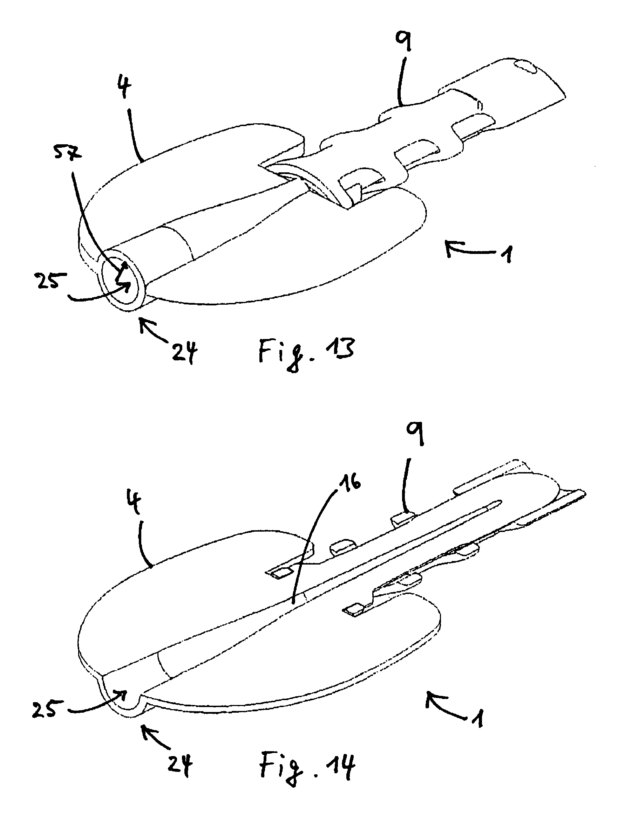

Figs. 13 and 14 illustrate a perspective and a sectional view of a slightly altered embodiment of the transfer device 1. Again, the transfer device 1 comprises the cannula 4 and the sheath 9, the latter being depicted in the closed position. The central lumen 16 of the cannula ends in the proximal opening 25 situated at the proximal end 24 of the cannula 4. In this embodiment the proximal opening 25 of the cannula 4 is configured as a female part of a Luer Taper connection. The proximal opening 25 has a round shape, an inner radius 57 of the opening 25 having a length of approximately 2 mm. The opening 25 of the central lumen 16 tapers at an angle of 6°.

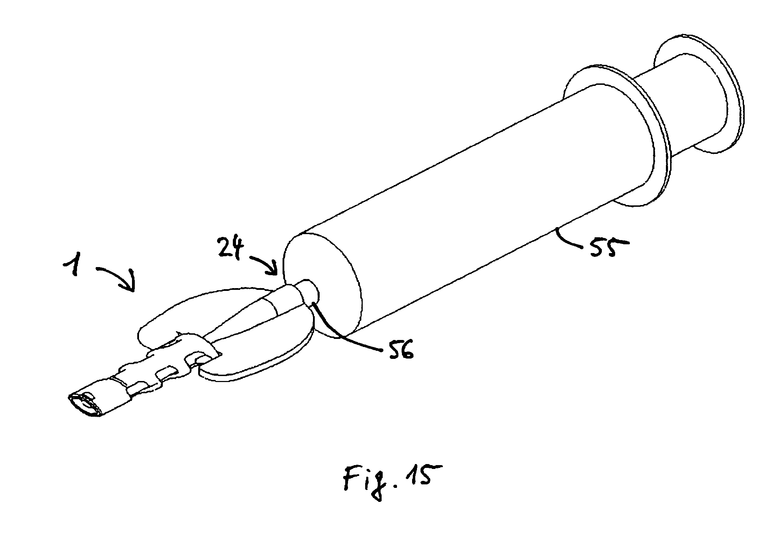

Fig. 15 shows the transfer device 1 from Figs. 13 and 14 connected to a plastic syringe 55. The syringe 55 features a male part 56 of a Luer Taper connection which is connected to the female Luer part formed by the proximal opening 25 at the proximal end 24 of the transfer device 1. In a similar manner, the transfer device 1 can be connected to any other device featuring a male Luer connector or an equivalent connector. Of course, the proximal opening 25 of the cannula 4 could equally well be configured as a male part of a Luer Taper connection. In the latter case, the cannula 4 could be connected to any component featuring a female Luer connector or a connector equivalent thereto.

## Claims

1. A transfer device (1) for delivering a medical fluid to a medical fluid container (3), the transfer device (1) comprising at least
- a cannula (4) with a blunt tip (5) formed at a distal end (6) of the cannula (4) and with a side opening (7) arranged at an axial distance (8) from the blunt tip (5) and
- a retractable sheath (9), the sheath (9) being configured to repeatedly expose and seal the side opening (7),
**characterised in that** the distal end section of the sheath (9) has an open front surface (10) for passing through at least a part of the cannula (4).

2. The transfer device (1) according to claim 1, **characterised in that** the cannula (4) comprises a plastic material.

3. The transfer device (1) according to any of the preceding claims, **characterised in that** the sheath (9) comprises an elastic material.

4. The transfer device (1) according to any of the preceding claims, **characterised in that** the sheath (9) has a tubular or hose-like shape, the sheath (9) preferably clasping around the cannula (4) when the sheath (9) seals the side opening (7).

5. The transfer device (1) according to any of the preceding claims, **characterised by** a mechanical stop (11) configured to prevent the sheath (9) from being pushed beyond the mechanical stop (11) along the axial direction of the cannula (4).

6. The transfer device (1) according to any of the preceding claims, **characterised in that** a stiffness of the sheath (9) causes the sheath (9) to seal the side opening (7) when no external forces act on the sheath (9).

7. The transfer device (1) according to any of the preceding claims, **characterised in that** the sheath (9) has perforations (15), the perforations (15) allowing the sheath (9) to fold at least partially when the sheath (9) exposes the side opening (7).

8. The transfer device (1) according to any of the preceding claims, **characterised in that** the cannula (4) has a non-circular cross section at least in a distal section (28) of the cannula (4) .

9. The transfer device (1) according to any of the preceding claims, **characterised in that** a cross-sectional area of a central lumen (16) of the cannula (4) amounts to less than 30 percent, preferably to less than 20 percent, more preferably to less than 10 percent of a cross-sectional area of the cannula (4), at least in a distal section (28) of the cannula (4).

10. The transfer device (1) according to any of the preceding claims, **characterised in that** the cannula (4) is at least partially coated with a lubricating coating.

11. The transfer device (1) according to any of the preceding claims, **characterised in that** a second opening of the cannula is configured as a male or a female part of a Luer Taper connection.

12. A transfer system (17) comprising the transfer device (1) according to any of the preceding claims and a bung (18) for sealing a medical fluid container (3), wherein the sheath (9) is configured to expose the side opening (7) of the cannula (4) upon insertion of the cannula (4) into an opening (19) of the bung (18) and to reseal the side opening (7) of the cannula (4) upon withdrawal of the cannula (4) from the opening (19) of the bung (18).

13. The transfer system (17) according to claim 12, **characterised in that** the blunt cannula (4) is incorporated into or connected to a tube barrel holder (20), the tube barrel holder (20) being configured to accommodate the fluid container (3) at least partially and to guide the cannula (4) into the opening (19) of the bung (18).

14. The transfer system (17) according to any of the claims 12 or 13, **characterised in that** the opening (19) of the bung (18) is designed as a self resealable and non-circular slit, the slit being configured to accommodate the cannula (4) at least partially and the bung (18) preferably tightly clasping around at least a part of the cannula (4) upon insertion of the cannula (4) into the slit.

15. The transfer system (17) according to any of the claims 12 to 14, **characterised in that** the sheath (9) comprises protuberances (21) at the distal end of the sheath (9) thereby increasing a contact surface between the sheath (9) and the bung (18) during insertion of the cannula (4) into the opening (19) of the bung (18), the bung (18) preferably having corresponding recesses (22) for accommodating the protuberances (21).
